(19)

**Europäisches Patentamt**
**European Patent Office**
**Office européen des brevets**

(11) **EP 3 222 344 A1**

(12) **EUROPÄISCHE PATENTANMELDUNG**

(43) Veröffentlichungstag:
**27.09.2017 Patentblatt 2017/39**

(51) Int Cl.:
**B01J 19/00** *(2006.01)*        **C07C 45/50** *(2006.01)*
**C07C 47/02** *(2006.01)*

(21) Anmeldenummer: **16161332.8**

(22) Anmeldetag: **21.03.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
• **HESS, Dieter**
  **45770 Marl (DE)**
• **GEILEN, Frank**
  **45721 Haltern am See (DE)**
• **FRANKE Robert**
  **45772 Marl (DE)**
• **TLATLIK, Stephen**
  **44309 Dortmund (DE)**
• **GOTTSCHALK, Axel**
  **45527 Hattingen (DE)**

(54) **KONTINUIERLICHES VERFAHREN ZUR HERSTELLUNG EINER CHEMISCHEN VERBINDUNG**

(57)    Die vorliegende Erfindung betrifft Verfahren zur Herstellung einer chemischen Verbindung in einer Reaktionsvorrichtung, die

- einen Reaktionssektor, der mindestens einen Reaktor umfasst,

- einen Abtrenn-und-Rückführ-Sektor, der mindestens eine Trenneinrichtung, Verbindungsrohrleitungen zur Leitung mindestens eines Reaktoraustrittsstroms zu der mindestens einen Trenneinrichtung und Verweileinrichtungen, die Verbindungsrohrleitungen zur Rückführung mindestens eines Rückführstroms von der mindestens einen Trenneinrichtung in den mindestens einen Reaktor umfassen, aufweist,

- mindestens eine Rohrleitung zur Zuführung von einem oder mehreren Zuführströmen, die mindestens einen Edukt- und optional mindestens einen Lösungsmittelstrom umfassen, in die Reaktionsvorrichtung, und

- mindestens eine Rohrleitung zur Ableitung mindestens eines Produktstroms aus der Trenneinrichtung aufweist, wobei mindestens ein Zuführstrom dem Abtrenn-und-Rückführ-Sektor zugeleitet wird sowie eine Mini-Plant zur Durchführung des Verfahrens.

Fig. 8

EP 3 222 344 A1

**Beschreibung**

Die vorliegende Erfindung betrifft ein kontinuierliches Verfahren zur Herstellung einer chemischen Verbindung insbesondere zur Optimierung von Prozessparametern in einem solchen Verfahren und eine Miniplant zur Verwendung in einem solchen Verfahren.

Aktuelle, kontinuierlich betriebene chemisch-genutzte (Miniplant-)Anlagen mit Recycleströmen führen Einsatzstoffe i.d.R. direkt dem Reaktor bzw. den Reaktoren zu. Die hier stattfindenden Reaktionen bilden die Produkte. Nach gegebener mittlerer Verweilzeit und entsprechend erfolgter Umsetzung wird ein Teil des Reaktionsgemisches kontinuierlich aus dem Reaktor abgezogen. Durch kontinuierliches Hinzufügen frischen Einsatzstoffes und/oder Zurückführung von Stoffströmen aus der Aufarbeitungssektion wird eine kontinuierliche Reaktionsführung ermöglicht. Der Flüssigkeitsfüllstand in den Reaktoren wird hierbei oftmals geregelt und bleibt somit weitgehend konstant.

Das aus den Reaktoren abgetrennte Produktgemisch wird in geeigneten Trennoperationen aufgearbeitet. Ziel ist es, die gebildeten Produkte zu isolieren und aus dem Prozess zu entfernen. Weiterhin sollen nicht umgesetzte Einsatzstoffe sowie verwendete Lösungsmittel und Hilfsstoffe möglichst umfassend in den Reaktionsprozess zurückgeführt werden.

Ein besonderes Augenmerk gilt hierbei häufig der Rückführung eines ggf. vorhandenen homogenverteilten Katalysators. Da dieser oftmals kostspielig ist und gesundheits- oder umweltrelevant sein kann, sind die Trennoperationen derart gewählt, dass der Katalysator vermindert oder bevorzugt nicht in den abgetrennten und ausgetragenen Produktphasen zu finden ist. Katalysatorverluste werden minimiert.

Der oder die verbleibenden Restströme, die neben nicht abgetrennten Komponenten ebenfalls den homogenen Katalysator enthalten können, werden in den Reaktor zurückgeführt.

Eine Veränderung der Betriebsparameter stellt bei der Prozessführung mit Recycleströmen häufig eine Herausforderung dar. Ursächlich hierfür ist, dass Ausgleichsprozesse innerhalb der Anlage notwendig sind, um nach erfolgter Änderung einen neuen gewünschten, oft stationären Betriebspunkt zu erreichen. Dies ist oftmals zeitintensiv, da sich die Änderung in einem Apparat oder Strom mit den Stoffströmen, die die verschiedenen Apparate der Reaktionssektion und der Recycle-&Separationssektion miteinander verbinden, durch die gesamte Anlage fortpflanzen muss, um den Ausgleich verschiedener Parameter innerhalb der Gesamtanlage zu ermöglichen. Aufgrund der seriellen Verkettung der Apparate und Rohrleitungen sind zum Erreichen des stationären Betriebs i.d.R. mehrere Umwälzungszyklen durch die gesamte Anlage unumgänglich.

Die Durchmischung der relevanten Phasen in den Rückhaltevolumina der Apparate erweist sich oftmals als unproblematisch, da hier eine gute Durchmischung stattfindet und somit Änderungen der Betriebsparameter aus vorgelagerten Apparaten schnell im entsprechenden Apparat realisiert werden können. Die Rohrleitungen andererseits werden i.d.R. pfropfenförmig durchströmt. Abgesehen von Diffusion, die gerade bei niedrigen Temperaturen eine untergeordnete Rolle spielt, kommt es in den Rohren nur zu einer vernachlässigbaren axialen Durchmischung. Eine Durchmischung und der Ausgleich von Änderungen können deshalb nur effizient erfolgen, wenn ein Stoffstromvolumenelement das Rohr vollständig durchströmt hat und die Durchmischung in einem folgenden Apparat, der besser durchmischt ist, stattfindet. Das Verhältnis aus Recyclestromflussrate und Rohrleitungsvolumen dominiert deshalb oft das Verhalten und die Geschwindigkeit eines Ausgleichsprozesses.

Gerade beim Betrieb von kontinuierlichen Miniplant-Anlagen ist der Zeitbedarf zum Erzielen eines annähernd vollständigen Ausgleichprozesses signifikant. Einerseits kennzeichnen sich Miniplant-Anlagen oftmals durch ein vergleichsweise weites Netz aus Rohrleitungen; damit bedingt sich ein vergleichsweise hohes Verweilzeitvolumen in den axial schlecht durchmischten Leitungen. Da andererseits die Flussraten der Stoffströme, die im Miniplant-Maßstab auftreten, üblicherweise relativ gering sind, können leicht mehr als 0.5 bis 2 Reaktionsverweilzeiten benötigt werden, um das Leitungsvolumen vollständig auszutauschen. Als Resultat der beschriebenen Phänomene können der Ausgleichprozess und das erneute Erreichen eines gewünschten Zustandes leicht mehr als 2 bis 10 Reaktorverweilzeiten betragen.

Hinzu kommt, dass Anlagen im Miniplant-Maßstab häufig eingesetzt werden, um im Rahmen von Forschung und Entwicklung Parameterstudien durchzuführen. Diese Untersuchungen zielen oft darauf ab, eine Vielzahl von stationären Betriebszuständen zu untersuchen, was wiederholt das Einstellen eines Gleichgewichts erfordern. Der signifikante Zeitbedarf der Ausgleichprozesse wirkt sich deshalb gerade im Miniplant-Betrieb besonders nachteilig aus.

Aufgabe der vorliegenden Erfindung ist es daher, ein eingangs beschriebenes Verfahren und eine dafür geeignete Miniplant zur Verfügung zu stellen, mit denen sich der Zeitbedarf von Ausgleichsprozessen bei Änderung des Betriebszustands eines Verfahrens bis zur Erreichung eines stationären Zustands minimieren lässt. Dieses Verfahren soll insbesondere für die Optimierung von Prozessparametern eines Verfahrens zur Herstellung einer chemischen Verbindung geeignet sein.

Diese Aufgabe wird durch ein kontinuierliches Verfahren zur Herstellung einer chemischen Verbindung in einer Reaktionsvorrichtung, die

- einen Reaktionssektor, der mindestens einen Reaktor umfasst,
- einen Abtrenn-und-Rückführ-Sektor, der mindestens eine Trenneinrichtung, Verbindungsrohrleitungen zur Leitung mindestens eines Reaktoraustrittsstroms zu der mindestens einen Trenneinrichtung und Verweileinrichtungen, die Verbindungsrohrleitungen zur Rückführung mindestens eines Rückführstroms von der mindestens einen Trenneinrichtung in den mindestens einen Reaktor umfassen, aufweist,
- mindestens eine Rohrleitung zur Zuführung von einem oder mehreren Zuführströmen, die mindestens einen Edukt- und optional mindestens einen Lösungsmittelstrom umfassen, in die Reaktionsvorrichtung, und
- mindestens eine Rohrleitung zur Ableitung mindestens eines Produktstroms aus der Trenneinrichtung

aufweist, wobei mindestens ein Zuführstrom dem Abtrenn-und-Rückführ-Sektor zugeleitet wird.

Insbesondere betrifft die vorliegende Erfindung ein Verfahren zur Optimierung der Prozessparameter eines Verfahrens, bei dem mindestens ein Prozessparameter systematisch variiert wird und ein Zielparameter in Abhängigkeit der eingestellten Prozessparameter gemessen wird und anhand der gemessenen Zielparameterwerte die optimale Prozessparametereinstellung ermittelt wird.

Geeignete Prozessparameter, die variiert werden können, können aus Temperatur, Druck, Eduktkonzentration im Reaktor, zu verwendendem Katalysator, Katalysatorkonzentration im Reaktor, Reaktorverweilzeit, Trenneinrichtungsverweilzeit und beliebigen Kombinationen davon ausgewählt sein.

Geeignete Zielparameter können aus Ausbeute an Wertprodukt, Selektivität für das Wertprodukt, Umsatz und Raum-Zeit-Ausbeute an Wertprodukt ausgewählt sein.

Weiterhin wird die oben definierte Aufgabe durch eine Miniplant zur Verwendung in dem erfindungsgemäßen Verfahren umfassend:

- einen Reaktionssektor, der mindestens einen Reaktor umfasst,
- einen Abtrenn-und-Rückführ-Sektor, der mindestens eine Trenneinrichtung, Verbindungsrohrleitungen zur Leitung mindestens eines Reaktoraustrittsstroms zu der mindestens einen Trenneinrichtung und Verweileinrichtungen, die Verbindungsrohrleitungen zur Rückführung mindestens eines Rückführstroms von der mindestens einen Trenneinrichtung in den mindestens einen Reaktor umfassen, aufweist,
- mindestens eine Rohrleitung zur Zuführung von einem oder mehreren Zuführströmen, die mindestens einen Edukt- und optional mindestens einen Lösungsmittelstrom umfassen, in die Reaktionsvorrichtung, und
- mindestens eine Rohrleitung zur Ableitung mindestens eines Produktstroms aus der Trenneinrichtung, wobei

mindestens eine der Rohrleitungen zur Zuführung von einem oder mehreren Zuführströmen mit einer der Einrichtungen des Abtrenn-und-Rückführ-Sektors verbunden ist, gelöst.

Anhand der Figuren soll die Erfindung näher erläutert werden. Hierfür zeigen:

Fig.1     zeigt ein Fließdiagramm eines Verfahrens nach dem Stand der Technik.
Fig.2     zeigt die Fließdiagramme zweier Ausführungsformen des erfindungsgemäßen Verfahrens.
Fig.3     zeigt das Simulationsfließbild des Vergleichsversuchs.
Fig.4     zeigt die Quantifizierung der Volumenströme während der Simulationszeit für den Vergleichsversuch.
Fig.5     zeigt den Verlauf der Katalysatorkonzentration während der Simulationszeit für den Vergleichsversuch im Reaktor, in der Trenneinheit und im Rückführstrom.
Fig.6     zeigt den Umsatz von Octen und die Ausbeute an n-Nonanal und an Nebenprodukten für den Vergleichsversuch.
Fig.7     zeigt die Menge an n-Nonanal produziert über die Simulationszeit für den Vergleichsversuch.
Fig.8     zeigt das Simulationsfließbild des erfindungsgemäßen Versuchs.
Fig.9     zeigt die Quantifizierung der Volumenströme während der Simulationszeit für den erfindungsgemäßen Versuch.
Fig.10     zeigt den Verlauf der Katalysatorkonzentration während der Simulationszeit für den erfindungsgemäßen Versuch im Reaktor, in der Trenneinheit und im Rückführstrom.

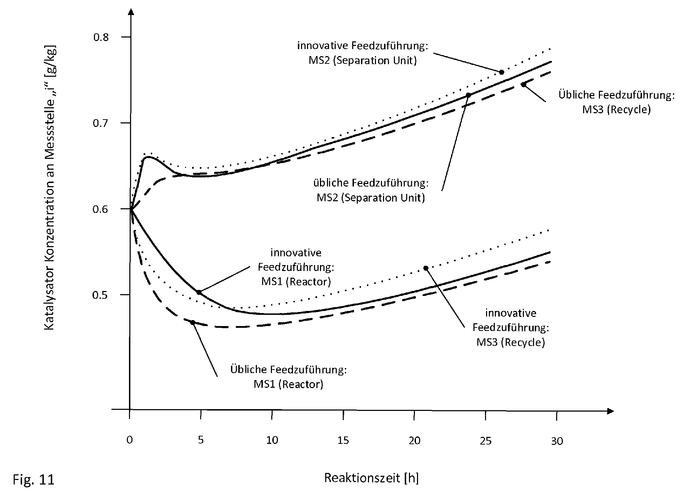

Fig.11     zeigt den Vergleich der Katalysatorkonzentrationen im Vergleichsversuch (gepunktete Linien) und der Katalysatorkonzentration im erfindungsgemäßen Versuch (solide Linien).
Fig.12     zeigt den Umsatz von Octen und die Ausbeute an n-Nonanal und an Nebenprodukten für den erfindungsgemäßen Versuch.
Fig.13     zeigt die Menge an n-Nonanal produziert über die Simulationszeit für den erfindungsgemäßen Versuch.

Um die in der Reaktionsvorrichtung stattfindenden Ausgleichsprozesse zu beschleunigen und somit schneller einen gewünschten, ggf. stationären Betriebszustand erreichen zu können, wurde eine verbesserte, innovative Zuleitungsstrategie für Zuführströme entwickelt. Im Gegensatz zur Zuführung nach dem Stand der Technik, die die Zuführströme direkt in die Reaktoren führt, werden bei der neuartigen Zuführungsstrategie die Zuführströme insbesondere, oder zumindest Teile davon, in den Abtrenn-und-Rückführ-Sektor zugeleitet. Hierdurch vergrößert sich die Flussrate des Rückführstromes oftmals signifikant, da nun neben den zurückgeführten Komponenten ebenfalls die Einsatzstoffe durch die Rückführrohrleitungen in die Reaktoren eingeleitet werden müssen. Das die Ausgleichsprozesse limitierende Volumen der Rohrleitungen des Abtrenn-und-Rückführ-Sektors wird mit einem höheren Flüssigkeitsdurchsatz durchströmt; der Stoffaustausch in der sich in den Rohrleitungen befindlichen Substanzmenge, der die Geschwindigkeit der Ausgleichsprozesses häufig dominiert, findet schneller statt. Somit werden die Ausgleichprozesse beschleunigt.

Um diesen Effekt möglichst umfassend und damit möglichst effizient nutzen zu können, erfolgt die Zuleitung bevorzugt möglichst weit stromaufwärts in dem Abtrenn-und-Rückführ-Sektor, damit ein maximales Rohrvolumen mit der erhöhten Flussrate durchströmt werden kann. Der mindestens eine Zuführstrom wird daher bevorzugt der Trenneinrichtung oder einer der Verweileinrichtungen zugeleitet. Die Verweileinrichtungen umfassen insbesondere Verbindungsrohrleitungen zur Rückführung mindestens eines Rückführstroms von der Trenneinrichtung in den Reaktor, Pumpen und falls notwendig Verweilzeitbehälter. Der Zuführstrom kann daher einer der Verbindungsrohrleitungen zur Rückführung mindestens eines Rückführstroms von der Trenneinrichtung in den Reaktor, vorzugsweise in Stromrichtung unmittelbar nach der Trenneinrichtung zugeleitet werden. Die Zuführung kann somit direkt nach den Trennoperationen erfolgen, oder, wenn die Trennoperationen nicht nachteilig beeinflusst werden, auch direkt in die Trenneinrichtung oder sogar vor dieser.

Es ist gemäß der vorliegenden Erfindung möglich, mehrere Zuführströme vor Zuleitung in den Abtrenn-und-Rückführ-Sektor miteinander zu kombinieren. Vorteilhafterweise können die Zuführströme mindestens einen Eduktstrom und mindestens einen Lösungsmittelstrom umfassen.

Das erfindungsgemäße Verfahren ist insbesondere für Flüssigphasenreaktionen geeignet. Somit kann der mindestens eine Zuführstrom, der mindestens eine Reaktoraustrittsstrom und/oder der mindestens eine Rückführstrom flüssig sein.

Das erfindungsgemäße Verfahren findet insbesondere bei katalytischen Prozessen in vorteilhafter Weise Anwendung. In diesem Fall enthält der mindestens eine Rückführstrom einen Katalysator, insbesondere einen in der flüssigen Phase des Rückführstroms löslichen Homogenkatalysator.

In einer bevorzugten Ausführungsform der vorliegenden Erfindung betrifft das Verfahren eine katalysierte Hydroformylierung, vorzugsweise unter Verwendung eines Iridiumkatalysators.

Wie aus den nachfolgenden Beispielen deutlich wird, beobachtet man bei der Simulierung eines katalytischen Prozesses z.B. die iridium-katalysierte Flüssigphasen-Hydroformylierung von Octen während der Simulationsdauer, zuerst eine Abreicherung und danach eine Anreicherung von Katalysator im Reaktor und eine Anreicherung des Katalysators in der Trenneinheit. Dies kann wie folgt erklärt werden:

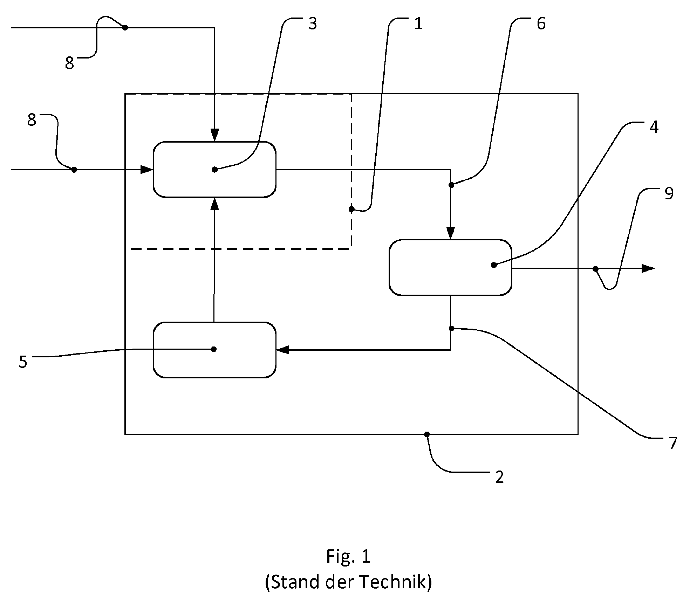

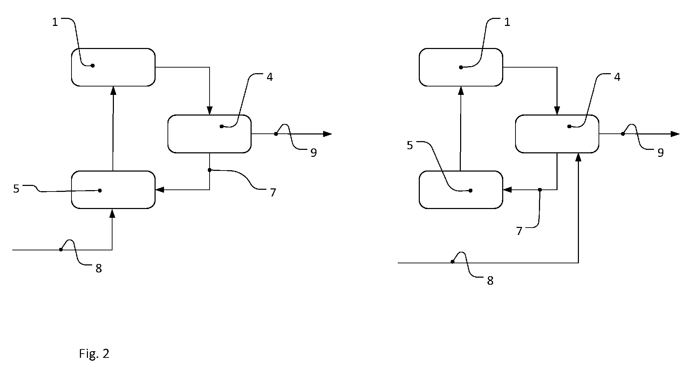

Der Unterschied zwischen einem Verfahren nach dem Stand der Technik und dem erfindungsgemäßen Verfahren wird in den Figuren 1 und 2 verdeutlicht. Dabei zeigt Fig. 1 skizzenhaft die Prozessführung gemäß Stand der Technik, während Fig. 2 zwei alternative Einsatzstoffzuführungen gemäß der vorliegenden Erfindung zeigt.

Hierbei werden folgende Bezugszeichen verwendet:

| | |
|---|---|
| Reaktionssektor | (1) |
| Abtrenn-und-Rückführ-Sektor | (2) |
| Reaktor | (3) |
| Trenneinrichtung | (4) |
| Verweileinrichtung | (5) |
| Verbindungsrohrleitung | (6)/(7) |
| Zuführleitung | (8) |
| Produktleitung | (9) |

Durch die durch das erfindungsgemäße Verfahren erzielte Beschleunigung der Ausgleichsprozesse bei Änderung von Prozessparametern können Prozessoptimierungen insbesondere in einer Miniplant wesentlich effizienter und somit schneller und kostengünstiger im Vergleich zum Stand der Technik durchgeführt werden. Darüber hinaus kann oftmals die Leistungsfähigkeit einer Anlage in Bezug auf die Ausbeute und Selektivität für das Wertprodukt gesteigert werden.

Die vorliegende Erfindung wird nun anhand von Beispielen verdeutlicht.

**Vergleichsbeispiel: Anfahren einer iridium-katalysierten Hydroformylierung im Miniplant-Maßstab, Prozessführung gemäß Stand der Technik**

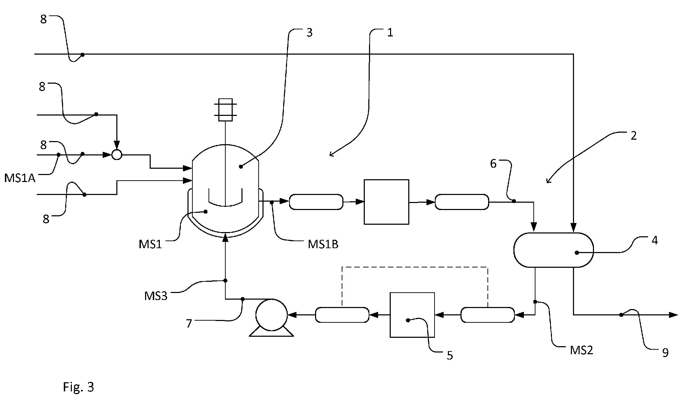

Um die beschriebenen Effekte zu zeigen und zu quantifizieren, wird eine homogen iridium-katalysierte Hydroformulierung von n-Octen dynamisch simuliert. Fig. 3 zeigt das Simulationsfließbild der Anlage. In Gegenwart des Katalysators setzt sich das flüssige n-Octen in der flüssigen Reaktionsphase des Reaktors (3) mit Kohlenstoffmonoxid (CO) und Wasserstoff (H$_2$) zum Wertprodukt n-Nonanal um. Neben der Hauptreaktion treten diverse Nebenreaktionen auf. Deshalb ist es wichtig, die Konzentration des Katalysators, der bevorzugt die Wertproduktbildung katalysiert, im Reaktor möglichst hoch zu halten, um eine hohe Selektivität bei guten Wertproduktausbeuten zu erreichen.

Zum Zeitpunkt des Anfahrens der Anlage, also zu Simulationsbeginn, werden Lösungsmittel, n-Octen, H$_2$ und CO sowie der Katalysator im Reaktor vorgelegt. Die Trennvorrichtung (4) und die Rohrleitungen (6, 7) sind ebenfalls mit Lösungsmittel und Katalysator gefüllt. Die Einsatzstoffe sowie die Makeup-Ströme zum Ersatz ausgetragenen Lösungsmittels werden kontinuierlich zugeleitet; ein Produktstrom wird kontinuierlich abgeführt.

Im Regelfall werden die Einsatzstoffe mit einer geringen Katalysatorkonzentration oftmals katalysatorfrei zugeführt. Eine geringe Katalysatorkonzentration bezeichnet eine Katalysatorkonzentration im einem Volumen oder einem Strom, die unterhalb der Konzentration eines Bezugsstromes oder-volumens liegt. Das Bezugsvolumen bildet i.d.R. eine Flüssigphase des Reaktors.

Um reaktionszeitabhängig die Veränderungen der Katalysatorkonzentration in den verschiedenen Anlagenteilen zu detektieren, werden fünf Messstellen "MS1A ... 3" vorgesehen. Fig. 3 zeigt die Verteilung der Messstellen in dem Fließbild. Die Messstellen sind im Einzelnen:

- MS1A: Volumenstrom des n-Octen
- MS1B: Volumenstrom des Produktstromes
- MS1: Katalysatorkonzentration im Reaktor
- MS2: Volumenstrom und Katalysatorkonzentration im Austrittsstrom der Trenneinheit
- MS3: Volumenstrom und Katalysatorkonzentration im Recyclestrom

Zu Reaktionsbeginn, dem Reaktionszeitpunkt $t_R$ = 0 h, ist die massenspezifische Katalysatorkonzentration in allen Apparaten und Prozessabschnitten gleich. Der volumenspezifische Zuführstrom des flüssigen n-Octens wird über die gesamte Betriebsperiode ebenso konstant gehalten wie die Flussrate des Rückführstroms. Eine Füllstandsregelung hält den Flüssigkeitsstand nach Erreichen der gewünschten Füllhöhe konstant. Überschüssige Flüssigkeit, die sich durch die kontinuierliche Zuführung von Zuführ- und Rückführstrom im Reaktor findet, wird als Produktstrom abgezogen und in die Trenneinrichtung (4) geleitet. Hier werden Teile der gebildeten Produkte aus dem Prozess entfernt; zusätzlich verlassen Teile nicht umgesetzter Einsatzstoffe sowie Hilfsstoffe den Prozess. Die Trennoperation ist hierbei derart gewählt, dass Katalysatorverluste durch Übergang in die abzutrennende Phase minimiert werden; in Übereinstimmung mit entsprechenden Experimenten wird angenommen, dass keine bzw. nur eine vernachlässigbare Menge an Katalysator den Prozess verlässt. Etwaige Verluste an Lösungsmitteln werden durch den Lösungsmittelzuführstrom ersetzt.

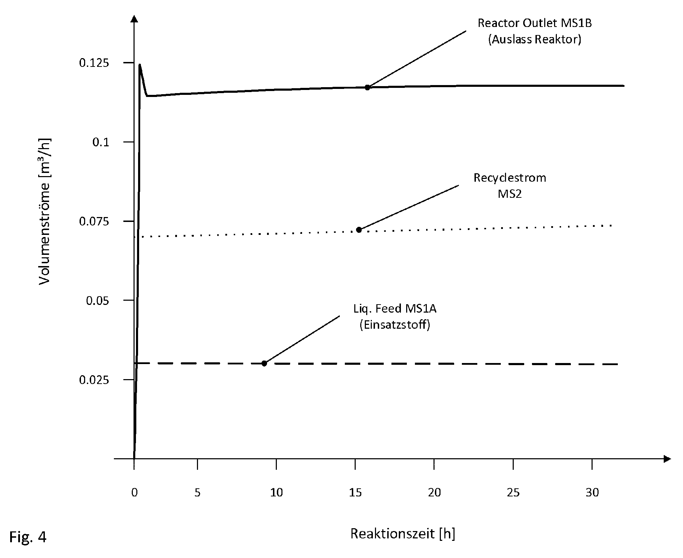

Fig. 4 zeigt den simulierten Verlauf der relevanten Volumenströme über eine Simulationsdauer von $t_R$ = 30 h.

Wie zuvor bereits bemerkt, ist die Katalysatorkonzentration in allen Prozessabschnitten zu Beginn der Simulation gleich. Mit fortschreitender Simulationsdauer zeigt es sich jedoch, dass diese Konzentration starken Veränderungen unterliegt. Die Katalysatorkonzentration im Reaktionsmedium fällt zunächst stark ab; nach etwa $t_R$ = 7,5 h ist ein Minimum erreicht. Danach steigt diese wieder an. Dementgegen erhöht sich die Konzentration der katalytisch aktiven Komponente in dem Abtrenn-und-Rückführ-Sektor (2); besonders in dem Rückführstrom nach der Trenneinrichtung (4).

Beide Effekte, die Abreicherung und spätere Anreicherung von Katalysator im Reaktor (3) sowie die Anreicherung des Katalysators in den Strömen in dem Abtrenn-und-Rückführ-Sektor (2) können erklärt werden. Um diese Abhängigkeiten verdeutlichen zu können, sei für eine vereinfachte Berechnung angenommen, dass die Dichte "$\rho$" der flüssigen Phase in allen Prozessabschnitten der Anlage gleich und konstant sei. Die Katalysatorkonzentration im Zuführstrom sei "$w_{Cat\_Feed}$ = 0". Unter Vernachlässigung eines Phasenübergangs verschiedener Komponenten kann folgende Bilanz aufgestellt werden:

$$V_{\mathrm{Re}c} * \rho = m_{\mathrm{Re}c}; V_{Feed} * \rho = m_{Feed}$$

$$V_{\mathrm{Pr}od} * \rho = m_{\mathrm{Pr}od} = m_{\mathrm{Re}c} + m_{Feed}; \text{ (maximaler Füllstand erreicht)}$$

$$m_{Cat_{Rec}} = m_{Rec} * w_{Cat_{Rec}}$$

$$m_{Cat_{Feed}} = m_{Feed} * w_{Cat_{Feed}} ; (hier : m_{Feed} * 0 = 0)$$

$$m_{Cat_{Prod}} = m_{Prod} * w_{Cat_{RCT}} = (m_{Rec} + m_{Feed}) * w_{Cat_{RCT}}$$

$$\frac{dm_{Cat_{RCT}}}{dt_R} = m_{Cat_{Rec}} + m_{Cat_{Feed}} - m_{Cat_{Prod}}$$

$$\frac{dm_{Cat_{RCT}}}{dt_R} = m_{Rec} * w_{Cat_{Rec}} + m_{Feed} * w_{Cat_{Feed}} - (m_{Rec} + m_{Feed}) * w_{Cat_{RCT}}$$

Allgemein gilt somit:

$$\frac{dm_{Cat_{RCT}}}{dt_R} = m_{Rec} * (w_{Cat_{Rec}} - w_{Cat_{RCT}}) + m_{Feed} * (w_{Cat_{Feed}} - w_{Cat_{RCT}})$$

Variable

m - Massenstrom [kg/h]; $\geq 0$
w - Massenkonzentration [kg/kg]; $\geq 0$
$t_R$ - Reaktionszeit [h]; $\geq 0$

Indices

Cat - Katalysator
RCT - Reaktionsgemisch betreffend
Rec - Rückführstrom betreffend
Feed - Zuführstromstrom betreffend
Prod - Reaktorausgangsstrom betreffend

Zum Reaktionszeitpunkt $t_R$ = 0 h liegt im Reaktor (2) dieselbe Katalysatorkonzentration wie im Rückführstrom vor. Letzterer wird in den Reaktor (2) gefördert. Zeitgleich tritt ein Zuführstrom in den Reaktor (2) ein, der keinen Katalysator oder zumindest eine im Vergleich zum Reaktionsgemisch geringere Katalysatorkonzentration enthält. Bedingt durch die gute bzw. ideale Durchmischung im Reaktor "verdünnt" der Zuführstrom mit geringer Katalysatorkonzentration das Reaktionsgemisch.

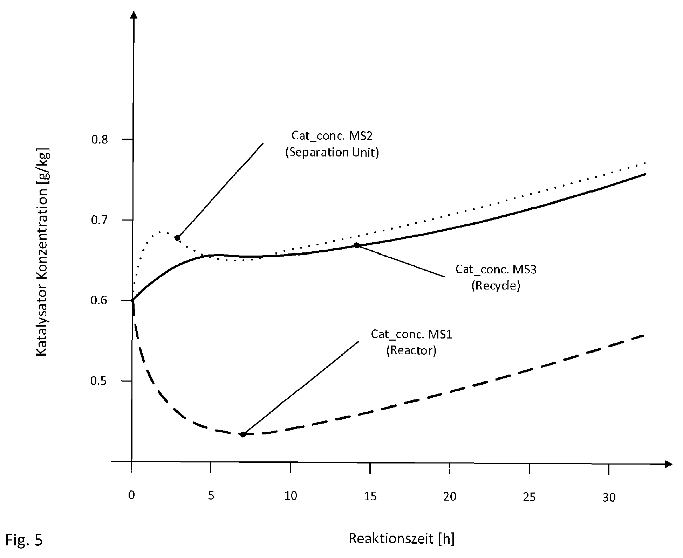

Zur Aufrechterhaltung eines konstanten Füllstandes wird ein Produktstrom kontinuierlich entnommen. Ist der maximale, geregelte Füllstand erreicht, entspricht die Flussrate des Produktstromes der Summe der Flussraten aus Rückführ- und Zuführstrom, ggf. modifiziert durch die stattfindenden Reaktionen. Da dieser Strom größer ist als der Rückführstrom und zu Reaktionsbeginn eine Katalysatorkonzentration enthält, die der des Reaktors - und allen anderen Prozessabschnitten - entspricht, wird absolut mehr Katalysator aus dem Reaktor ausgetragen als durch den Rückführstrom eingetragen wird. Die Konzentration an Katalysator im Reaktor (2) sinkt; die in Fig. 5 zu sehende Abreicherung des Katalysators im Reaktionsgemisch tritt nachhaltig auf.

Zu Reaktionsbeginn $t_R$ = 0 h ist die Konzentration des Katalysators in allen Prozessabschnitten gleich. Die Konzentration des Katalysators im n-Octen-Zuführstrom wird zu Null gesetzt. Es gilt:

$$w_{Cat_{RCT}} = w_{Cat_{Rec}} ; w_{Cat_{Feed}} = 0$$

Die gezeigten Gleichungen vereinfachen sich:

$$\frac{dm_{Cat_{RCT}}}{dt_R} = m_{\mathrm{Re}c} * (w_{Cat_{RCT}} - w_{Cat_{RCT}}) + m_{Feed} * (0 - w_{Cat_{RCT}})$$

$$\frac{dm_{Cat_{RCT}}}{dt_R} = m_{Feed} * (-w_{tR=0})$$

Wie das in letzter Gleichung auftretende negative Vorzeichen deutlich zeigt, muss die Katalysatorkonzentration im Reaktor unter Startbedingungen zunächst abnehmen. Der Produktstrom hingegen, der den Katalysator aus dem Reaktor abführt, wird der Aufarbeitung zugeführt. In der Trenneinrichtung (4) werden Teile des Produktstromes, nicht aber der Katalysator, selektiv aus dem Prozess entfernt. Da die Katalysatormenge im Strom vollständig erhalten bleibt, aber die Masse anderer Komponenten abnimmt, steigt die Katalysatorkonzentration im Strom, der die Trennsequenz verlässt, stark an.

Durch das Einleiten des aufgearbeiteten Produktstromes in die Rohrleitungen (7) des Rückführstroms wird ein äquivalentes, am Reaktor (2) anstehendes Volumenelement des Rückführstroms in den Reaktor verdrängt. Da allerdings keine axiale Durchmischung in den Rohren bedingt durch das pfropfenförmige Strömungsverhalten stattfindet, tritt dieser Strom mit der zunächst voreingestellten Katalysatorstartkonzentration in den Reaktor (2), wodurch sich die Abreicherung der Katalysatorkonzentration im Reaktor (2) gemäß dem oben beschriebenen Mechanismus fortsetzt.

Da die Konzentration des Katalysators im Reaktor (2) gefallen ist, verfügt der Rückführstrom bereits unter Bedingungen kurz nach Simulationsbeginn über eine Katalysatorkonzentration, die diejenige im Reaktionsgemisch übersteigt. Dadurch kann der Verdünnungseffektes durch den Eintrag weiterer Einsatzstoffes mit geringer Katalysatorkonzentration teilweise kompensiert werden. Die Rate der Abreicherung nimmt somit ab. Die Differenz der Konzentration an katalytisch aktiver Substanz zwischen dem Rückführstrom und dem Reaktionsgemisch reicht zu diesem jedoch noch nicht aus, um die Abreicherung vollständig zu stoppen oder umzukehren.

$$\frac{dm_{Cat_{RCT}}}{dt_R} = m_{\mathrm{Re}c} * (w_{Cat_{\mathrm{Re}c}} - w_{Cat_{RCT}}) + m_{Feed} * (w_{Cat_{Feed}} - w_{Cat_{RCT}})$$

$$w_{Cat_{\mathrm{Re}c}} > w_{Cat_{RCT}} ; m_{\mathrm{Re}c} * (w_{Cat_{\mathrm{Re}c}} - w_{Cat_{RCT}}) < \left| m_{Feed} * (w_{Cat_{Feed}} - w_{Cat_{RCT}}) \right|$$

Ab einem bestimmten Zeitpunkt, hier $t_R$ = 7,5 h, ist die Konzentration durch die beschriebenen Mechanismen in dem Rückführstrom derart angewachsen bzw. im Reaktionsgemisch derart gefallen, dass mehr Katalysator in den Reaktor (2) eingetragen wird als ausgetragen. Die Katalysatorkonzentration im Reaktionsmedium steigt an.

$$\frac{dm_{Cat_{RCT}}}{dt_R} = m_{\mathrm{Re}c} * (w_{Cat_{\mathrm{Re}c}} - w_{Cat_{RCT}}) + m_{Feed} * (w_{Cat_{Feed}} - w_{Cat_{RCT}})$$

$$w_{Cat_{\mathrm{Re}c}} \gg w_{Cat_{RCT}} ; m_{\mathrm{Re}c} * (w_{Cat_{\mathrm{Re}c}} - w_{Cat_{RCT}}) > \left| m_{Feed} * (w_{Cat_{Feed}} - w_{Cat_{RCT}}) \right|$$

Natürlicherweise nimmt mit zunehmender Konzentration des Katalysators im Reaktor (2) die Konzentration dieses im Rückführstrom zunächst ebenfalls zu, da ein Produktstrom mit einer erhöhten Katalysatorkonzentration aus dem Reaktionsgefäß entfernt wird.

Zu einem späteren, in Fig. 5 nicht mehr erfassten Zeitpunkt sind stationäre Bedingungen erreicht. Die Konzentrationsdifferenz zwischen Rückführstrom und Reaktionsgemisch ist gerade so groß, dass der Katalysatoreintrag per Rückführstrom die Verdünnung durch die katalysatorfreie Zuleitung des Zuführstroms sowie den vermehrter Abzug durch den Produktstrom kompensieren kann. Ein neuer stationärer Punkt ist erreicht.

$$\frac{dm_{Cat_{RCT}}}{dt_R} = m_{\text{Rec}} * \left( w_{Cat_{\text{Rec}}} - w_{Cat_{RCT}} \right) + m_{Feed} * \left( w_{Cat_{Feed}} - w_{Cat_{RCT}} \right)$$

$$w_{Cat_{\text{Rec}}} \gg w_{Cat_{RCT}} \, ; m_{\text{Rec}} * \left( w_{Cat_{\text{Rec}}} - w_{Cat_{RCT}} \right) = \left| m_{Feed} * \left( w_{Cat_{Feed}} - w_{Cat_{RCT}} \right) \right|$$

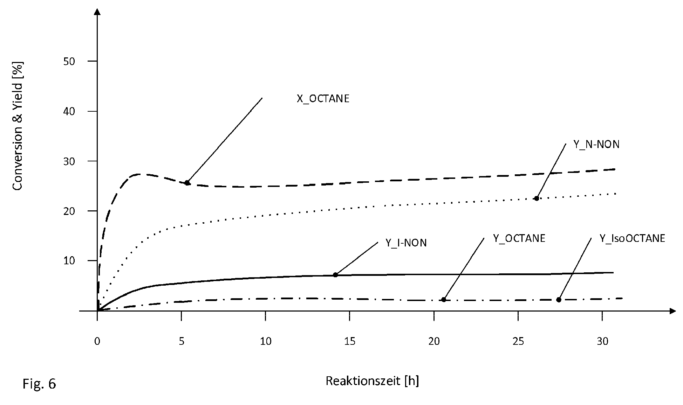

Unter der vereinfachten Annahme einer linearen Abhängigkeit der Produktionsrate des Wertproduktes n-Nonanal (N-NON) von der Katalysatorkonzentration im Reaktionsgemisch ergibt sich ein Produkt-Ausbeute-Verlauf, der in Fig. 6 gezeigt ist. Innerhalb der Simulationsdauer von 30 Stunden werden in der Miniplant mit konventioneller Feedzuleitung etwa 0,9 kmol des Zielproduktes erzeugt.

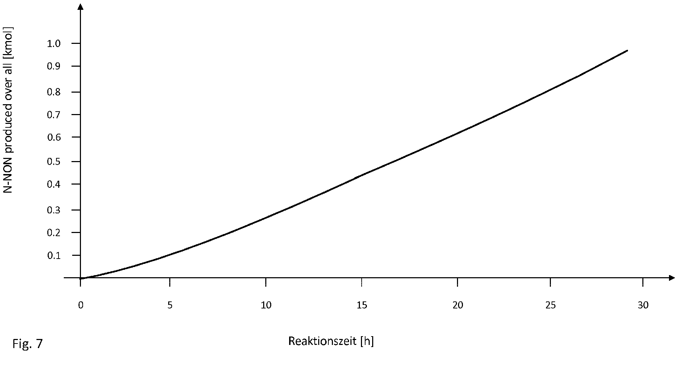

Fig. 6 und Fig. 7 zeigen die simulierten Verläufe von Umsatz und Ausbeuten sowie die Produktionsmenge des Wertproduktes gegen die Simulationszeit.

**Erfindungsgemäßes Beispiel "Anfahren einer iridium-katalysierten Hydroformylierung im Miniplant-Maßstab, erfindungsgemäße Prozessführung"**

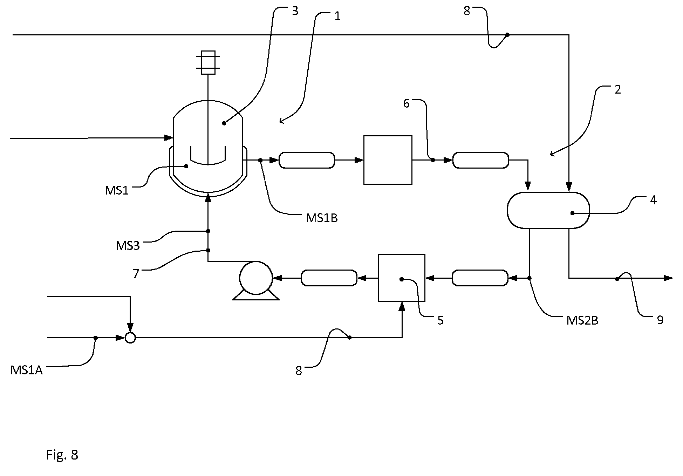

Analog zur Untersuchung der Anlage mit Zuführung gemäß Stand der Technik wird eine weitere dynamische Simulation des Prozesses mit erfindungsgemäßer Zuführung in die Recycle-&Separations-Sektion durchgeführt. Fig. 8 zeigt das Simulationsfließbild des erfindungsgemäßen Verfahrens inklusive Messstellen.

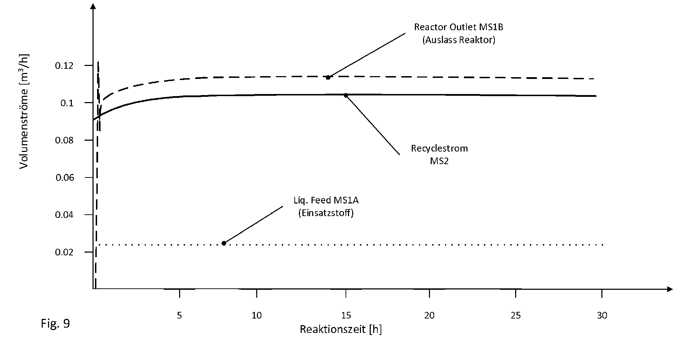

Alle Prozessparameter, Betriebsbedingungen und Abläufe entsprechen den Bedingungen im zuvor diskutierten Vergleichsbeispiel. Ausnahmen hiervon bilden der Zuleitungsort des Einsatzstoffe n-OCTEN und des Makeup-Stromes des Lösungsmittels sowie Anpassung der Flussrate des Rückführstroms, da dieser nun die Summe aus Rückführstrom und Zuführströmen realisieren muss. Fig. 9 verdeutlicht dies: bei gleicher Zuführflussrate wie im Falle des Vergleichsversuchs hat sich die Rückführstromflussrate um die Summe der externen Zuführflussraten erhöht. Folglich muss die Rückführstromrate abzüglich der Einsatzstoff- und Makeup-Ströme der Flussrate dieses Stromes im Vergleichsversuch entsprechen.

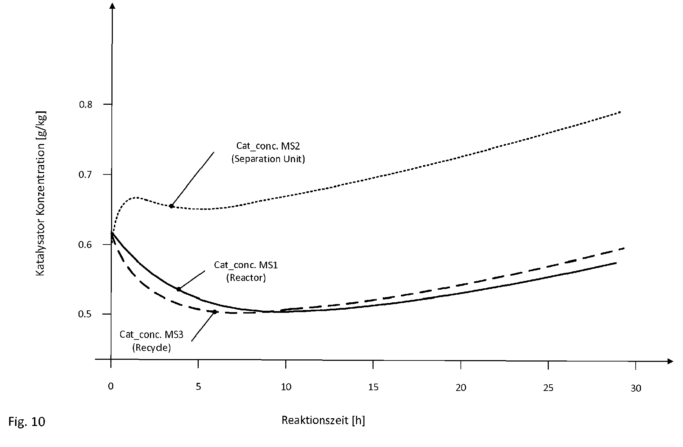

Fig. 10 zeigt den Verlauf der Katalysatorkonzentration an den gewählten Messstellen des erfindungsgemäßen Verfahrens. Ein ähnlicher Verlauf wie bei dem Vergleichsversuch ist erkennbar bzgl. der Konzentration des Katalysators im Reaktor als auch im Strom, der der Trenneinrichtung (4) entnommen wird. Bedingt durch die nun frühzeitig stattfindende Verdünnung des Rückführstroms durch die Zumischung der Zuführströme mit geringerer Katalysatorkonzentration sinkt die Konzentration des Katalysators im Rückführstrom, der dem Reaktor (3) zugeführt wird (MS3), zunächst ab.

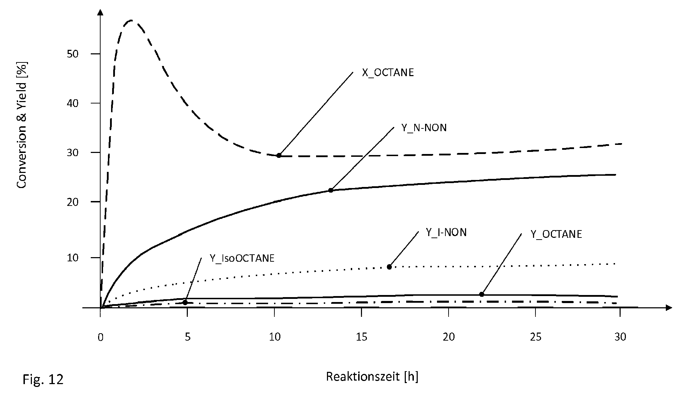

Vergleicht man die Katalysatorkonzentration, die sich bei dem Vergleichsversuch und bei dem erfindungsgemäßen Versuch ergibt, direkt miteinander (Fig. 12), wird der positive Effekt der Erfindung deutlich.

Verschiedene Effekte können erkannt werden: der Vergleich der Katalysatorkonzentrationen in den Reaktoren (3) zeigt, dass durch die geänderte, neuartige Einsatzstoffzuführung die Abnahme der Katalysatorkonzentration im Reaktionsgemisch deutlich langsamer stattfindet. Das Minimum der Konzentration wird erfindungsgemäß bei höheren Katalysatorkonzentrationen erreicht, was den verbesserten Ausgleichsprozess zeigt. Bedingt durch den geänderten Verlauf der Konzentration im Rückführstrom wird erfindungsgemäß dieses Minimum erst deutlich später als im Vergleichsversuch erreicht; jedoch ist zu sehen, dass die Konzentration des Katalysators im Reaktor (3) im erfindungsgemäßen Beispiel stets höher ist als die Konzentration im Vergleichsbeispiel. Dadurch ergeben sich bei dem erfindungsgemäßen Verfahren höhere Wertproduktreaktionsraten. Dies verbessert somit die Leistungsfähigkeit des Gesamtprozesses.

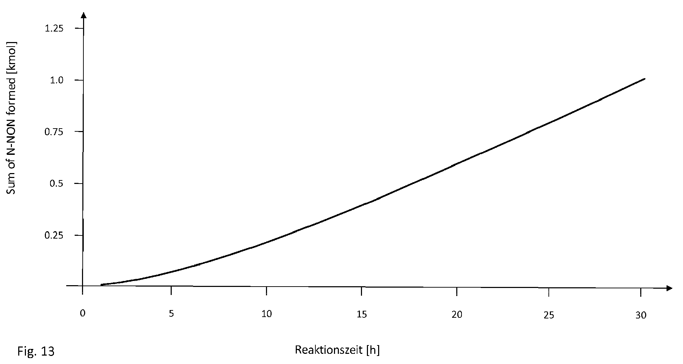

Fig. 12 und 13 zeigen die verbesserte Prozessleistung. Im Gegensatz zum Vergleichsbeispiel, bei dem der Umsatz an n-Octen über 30 h stets unterhalb 30 % liegt (Fig. 6), unterschreitet das erfindungsgemäße Verfahren diese Schwelle ab ca. $t_R > 0,5$ h nicht. Es werden sogar deutliche größere Werte erreicht. Im Vergleichsversuch werden $t_R = 12,5$ h benötigt (Fig. 6) um eine Ausbeute an n-Nonanal von 20 % zu erreichen; das erfindungsgemäße Verfahren durchbricht diesen Wert bereits nach $t_R = 10$ h. Der beschleunigte Ausgleichsprozess wird erneut deutlich.

Bedingt durch diese positive Veränderung des Anlagenbetriebs im erfindungsgemäßen Beispiel wird eine gesteigerte Menge an Wertprodukt produziert. Im Vergleich zum Vergleichsbeispiel, das etwa 0.9 kmol n-Nonanal in 30 h generiert (Fig.7) produziert das Vergleichsbeispiel ca. 1,05 kmol des Wertproduktes, was einer Mengensteigerung von 16,7 % entspricht.

Somit belegt der Vergleich zwischen Vergleichsbeispiel und erfindungsgemäßem Beispiel die oben diskutierten Vorteile der vorliegenden Erfindung.

**Patentansprüche**

1.  Kontinuierliches Verfahren zur Herstellung einer chemischen Verbindung in einer Reaktionsvorrichtung, die

    - einen Reaktionssektor (1), der mindestens einen Reaktor (3) umfasst,
    - einen Abtrenn-und-Rückführ-Sektor (2), der mindestens eine Trenneinrichtung (4), Verbindungsrohrleitungen (6) zur Leitung mindestens eines Reaktoraustrittsstroms zu der mindestens einen Trenneinrichtung (4) und Verweileinrichtungen (5), die Verbindungsrohrleitungen (7) zur Rückführung mindestens eines Rückführstroms von der mindestens einen Trenneinrichtung (4) in den mindestens einen Reaktor (1) umfassen, aufweist,
    - mindestens eine Rohrleitung (8) zur Zuführung von einem oder mehreren Zuführströmen, die mindestens einen Edukt- und optional mindestens einen Lösungsmittelstrom umfassen, in die Reaktionsvorrichtung, und
    - mindestens eine Rohrleitung (9) zur Ableitung mindestens eines Produktstroms aus der Trenneinrichtung (4)

    aufweist, wobei mindestens ein Zuführstrom dem Abtrenn-und-Rückführ-Sektor (2) zugeleitet wird.

2.  Verfahren nach Anspruch 1, wobei der mindestens eine Zuführstrom der Trenneinrichtung (4) oder einer der Verweileinrichtungen (5) zugeleitet wird.

3.  Verfahren nach einem der vorstehenden Ansprüche, wobei der mindestens eine Zuführstrom einer Verbindungsrohrleitung (7), vorzugsweise in Stromrichtung unmittelbar nach der mindestens einen Trenneinrichtung (4) zugeleitet wird.

4.  Verfahren nach einem der vorstehenden Ansprüche, wobei mehrere Zuführströme vor Zuleitung in den Abtrenn-und-Rückführ-Sektor (2) miteinander kombiniert werden.

5.  Verfahren nach einem der vorstehenden Ansprüche, wobei die Zuführströme mindestens einen Eduktstrom und mindestens einen Lösungsmittelstrom umfassen.

6.  Verfahren nach einem der vorstehenden Ansprüche, wobei der mindestens eine Zuführstrom, der mindestens eine Reaktoraustrittsstrom und/oder der mindestens eine Rückführstrom flüssig ist.

7.  Verfahren nach einem der vorstehenden Ansprüche, wobei der mindestens eine Rückführstrom einen Katalysator enthält.

8.  Verfahren nach Anspruch 7, wobei der Rückführstrom flüssig ist und der Katalysator ein Homogenkatalysator ist.

9.  Verfahren nach einem der vorstehenden Ansprüche, wobei das Verfahren eine katalysierte Hydroformylierung ist, vorzugsweise unter Verwendung eines Iridiumkatalysators.

10. Verfahren nach einem der vorstehenden Ansprüche, wobei die Reaktionsvorrichtung eine Miniplant ist.

11. Verfahren nach einem der vorstehenden Ansprüche, insbesondere nach Anspruch 10, wobei das Verfahren ein Verfahren zur Optimierung der Prozessparameter des Verfahrens ist, bei dem mindestens ein Prozessparameter systematisch variiert wird und ein Zielparameter in Abhängigkeit der eingestellten Prozessparameter gemessen wird und anhand der gemessenen Zielparameterwerte die optimale Prozessparametereinstellung ermittelt wird.

12. Verfahren nach Anspruch 11, wobei der Prozessparameter aus Temperatur, Druck, Eduktkonzentration im Reaktor, zu verwendendem Katalysator, Katalysatorkonzentration im Reaktor, Reaktorverweilzeit, Trenneinrichtungsverweilzeit und beliebigen Kombinationen davon ausgewählt ist.

13. Verfahren nach einem der Ansprüche 11 oder 12, wobei der Zielparameter aus Ausbeute an Wertprodukt, Selektivität für das Wertprodukt, Umsatz und Raum-Zeit-Ausbeute an Wertprodukt ausgewählt ist.

14. Miniplant zur Verwendung in einem Verfahren nach einem der Ansprüche 1 bis 13 umfassend:

    - einen Reaktionssektor (1), der mindestens einen Reaktor (3) umfasst,
    - einen Abtrenn-und-Rückführ-Sektor (2), der mindestens eine Trenneinrichtung (4), Verbindungsrohrleitungen (6) zur Leitung mindestens eines Reaktoraustrittsstroms zu der mindestens einen Trenneinrichtung (4) und Verweileinrichtungen (5), die Verbindungsrohrleitungen (7) zur Rückführung mindestens eines Rückführstroms von der mindestens einen Trenneinrichtung (4) in den mindestens einen Reaktor (1) umfassen, aufweist,
- mindestens eine Rohrleitung (8) zur Zuführung von einem oder mehreren Zuführströmen, die mindestens einen Edukt- und optional mindestens einen Lösungsmittelstrom umfassen, in die Reaktionsvorrichtung, und
- mindestens eine Rohrleitung (9) zur Ableitung mindestens eines Produktstroms aus der Trenneinrichtung (4), wobei

mindestens eine der Rohrleitungen (8) zur Zuführung von einem oder mehreren Zuführströmen mit einer der Einrichtungen (4, 5, 6, 7) des Abtrenn-und-Rückführ-Sektors (2) verbunden ist.

15. Miniplant nach Anspruch 14, wobei mindestens eine der Rohrleitungen (8) zur Zuführung von einem oder mehreren Zuführströmen mit der Trenneinrichtung (4) oder einer der Verweileinrichtungen (5) verbunden ist.

16. Miniplant nach Anspruch 15, wobei mindestens eine der Rohrleitungen (8) zur Zuführung von einem oder mehreren Zuführströmen mit einer Verbindungsrohrleitung (7), vorzugsweise in Stromrichtung unmittelbar nach der mindestens einen Trenneinrichtung (4) verbunden ist.

Fig. 1
(Stand der Technik)

Fig. 2

Fig. 3

Fig. 4

Fig. 5

Fig. 6

Fig. 7

Reaktionszeit [h]

Fig. 8

Fig. 9

Fig. 10

innovative Feedzuführung:
MS2 (Separation Unit)

Übliche Feedzuführung:
MS3 (Recycle)

übliche Feedzuführung:
MS2 (Separation Unit)

innovative
Feedzuführung:
MS1 (Reactor)

innovative
Feedzuführung:
MS3 (Recycle)

Übliche Feedzuführung:
MS1 (Reactor)

Katalysator Konzentration an Messstelle „i" [g/kg]

Reaktionszeit [h]

Fig. 11

Fig. 12

Fig. 13

**Europäisches Patentamt**
**European Patent Office**
**Office européen des brevets**

## EUROPÄISCHER RECHERCHENBERICHT

Nummer der Anmeldung

EP 16 16 1332

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2013/156600 A1 (VITO [BE]) 24. Oktober 2013 (2013-10-24) | 1-8, 10-16 | INV. B01J19/00 C07C45/50 |
| A | * Seite 19, Zeile 30 - Seite 28, Zeile 7; Anspruch 1; Abbildungen 1,2 * ----- | 9 | |
| A | WO 2015/071113 A1 (BASF SE [DE]) 21. Mai 2015 (2015-05-21) * Ansprüche; Abbildung 1 * ----- | 1-16 | ADD. C07C47/02 |
| A | WO 2007/036424 A1 (EVONIK OXENO GMBH [DE]) 5. April 2007 (2007-04-05) * Ansprüche; Abbildungen 1-5; Tabellen * ----- | 1-16 | |

**RECHERCHIERTE SACHGEBIETE (IPC)**

B01J
C07C

Der vorliegende Recherchenbericht wurde für alle Patentansprüche erstellt

| Recherchenort | Abschlußdatum der Recherche | Prüfer |
|---|---|---|
| Den Haag | 9. September 2016 | Ginoux, Claude |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...............................................................................
& : Mitglied der gleichen Patentfamilie, übereinstimmendes
    Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 16 16 1332

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-09-2016

| Im Recherchenbericht angeführtes Patentdokument | Datum der Veröffentlichung | Mitglied(er) der Patentfamilie | Datum der Veröffentlichung |
|---|---|---|---|
| WO 2013156600 A1 | 24-10-2013 | AU 2013251065 A1 | 02-10-2014 |
| | | CA 2866661 A1 | 24-10-2013 |
| | | CN 104245096 A | 24-12-2014 |
| | | EP 2838643 A1 | 25-02-2015 |
| | | JP 2015514576 A | 21-05-2015 |
| | | RU 2014145482 A | 10-06-2016 |
| | | SG 11201406002Q A | 30-10-2014 |
| | | US 2015050706 A1 | 19-02-2015 |
| | | WO 2013156600 A1 | 24-10-2013 |
| WO 2015071113 A1 | 21-05-2015 | CN 105722813 A | 29-06-2016 |
| | | EP 3071542 A1 | 28-09-2016 |
| | | KR 20160089405 A | 27-07-2016 |
| | | WO 2015071113 A1 | 21-05-2015 |
| WO 2007036424 A1 | 05-04-2007 | AT 419917 T | 15-01-2009 |
| | | CN 101272860 A | 24-09-2008 |
| | | DE 102005046250 A1 | 29-03-2007 |
| | | EP 1931472 A1 | 18-06-2008 |
| | | ES 2320698 T3 | 27-05-2009 |
| | | JP 5091142 B2 | 05-12-2012 |
| | | JP 2009513320 A | 02-04-2009 |
| | | KR 20080053476 A | 13-06-2008 |
| | | TW I418396 B | 11-12-2013 |
| | | US 2008251456 A1 | 16-10-2008 |
| | | WO 2007036424 A1 | 05-04-2007 |

EPO FORM P0461

Für nähere Einzelheiten zu diesem Anhang : siehe Amtsblatt des Europäischen Patentamts, Nr.12/82